# EUROPEAN PATENT APPLICATION

(11) **EP 4 119 220 A1**
(43) Date of publication of application: **18.01.2023**
(21) Application number: 21186166.1
(22) Date of filing: 16.07.2021
(51) Int. Cl.: B01J 13/04, A61K 9/127

(54) **DEVICE AND METHOD FOR THE FORMATION OF ORGANIC NANOPARTICLES**

(71) Applicant: Jahn, Andreas, 8049 Zürich (CH)
(72) Inventor: Jahn, Andreas, 8049 Zürich (CH)
(74) Representative: Braun, André jr.

(57) **Abstract**

The present invention relates to a device (100, 200, 300, 400) for the formation of organic nanoparticles, comprising a cylindrical liquid feed line (14) and a first annular liquid feed line (24), wherein the cylindrical feed line (14) and the first annular feed line (24) are coaxially arranged and the first annular feed line (24) extends around the cylindrical feed line (14), the device comprising a first inlet (12) for the introduction of a first liquid into the cylindrical feed line (14) and a second inlet (22) for the introduction of a second liquid into the first annular feed line (24), wherein the device (100, 200, 300, 400) comprises a second annular feed line (34) and a third inlet (32) for the introduction of a third liquid into the second annular feedline (34), wherein the second annular feed line (34) is coaxially arranged with the cylindrical feed line (14) and the first annular feed line (24), and wherein the second annular feed line extends around and beyond the cylindrical feed line (14) and the first annular feed line (24). The present invention relates also to a method for the formation of organic nanoparticles.

## Description

### Technical Field

The present invention relates to the field of organic nanoparticles formation, in particular in the field of devices and methods for the formation of organic nanoparticles, especially liposomes vesicles.

### Background of the invention

Liposomes were first reported by Bangham in 1964 (A.D. Bangham, M.M. Standish, and J.C. Watkins. Diffusion of univalent ions across the lamellae of swollen phospholipids. Journal of Molecular Biology, 238-252 (1965)), and the first liposomal approved product in the market was approved in 1990 (Shah, Dhawan, et al.. Liposomes: Advancements and innovation in the manufacturing process. Advanced Drug Delivery Review, 102-122 (2020)). Liposomes, i.e., lipid bilayer vesicle nanoparticles, are formed when phospholipids and their derivatives are dispersed in a polar aqueous solution (e.g., water, buffer etc.). Encompassing the ability to encapsulate aqueous solutions within their core, isolate lipophilic compounds within their lipid bilayer, and support tailored surface chemistries for targeted delivery, liposomes are versatile, multifunctional nanoparticles with excellent biocompatibility. The versatility and advantages of liposomes as drug delivery systems for small molecules, peptides, genes, and monoclonal antibodies, as vaccine adjuvants, antibiotics, and anesthetic compounds is well studied and acknowledged in the scientific literature.

The in-vivo bioavailability of liposomes may exhibit a strong dependence on key parameters such as size, polydispersity and geometry/shape of the liposome impacting the therapeutic index of liposomal drugs. Smaller liposomes are known to exhibit slower blood clearance rate, thereby increasing drug availability. The ideal size for cancer-targeting liposomal nanotechnologies is commonly believed to be about 100 nm, which is thought to be large enough to provide a high drug payload volume while small enough to pass through leaky endothelial junctions in tumor tissues (US 10,434065 B2). In some studies, liposomes larger than 300 nm were not effectively taken up by cells in vitro, while smaller 100 nm liposomes exhibited rapid endocytosis. In other studies, 100 nm liposomes were found to maximize uptake into monocytes and macrophages compared with larger vesicles.

Thus, a challenge to liposomal drug delivery technologies has been the effective control over vesicle size during synthesis. Despite their widespread research, it is well recognized that the current processes used for manufacturing of liposomes suffers from many severe problems, including: i) multi-step batch processes; ii) the need for particle size reduction (often involving specialized tools and equipment such as extrusion and high-pressure homogenization) and iii) limited batch sizes. These limitations drive costs upwards, limit production and hinders development of liposomes.

Traditional liposome preparation is based on demanding bulk-scale batch processes such as film hydration, reverse phase evaporation, detergent depletion, emulsification, supercritical phase formation, and solvent injection technique associated with heterogeneous and poorly controlled chemical and mechanical conditions during lipid vesicle formation, resulting in broad vesicle size distribution. Additional post-processing steps such as membrane extrusion, homogenization, or sonication are thus required to regulate the size and reduce the polydispersity of the final vesicle suspensions. Moreover, even after repeated processing steps utilizing bulk techniques, such as sequential membrane extrusion or size exclusion chromatography, the resulting vesicles tend to be relatively large (>100 nm) and polydisperse. High energy dissipating post-processing steps such as sonication, while fast may be incompatible with heat labile drugs due to heat generation during the process.

In addition, conventional bulk synthesis and encapsulation methods often lead to significant active drug compound loss with waste that can approach 98 % for hydrophilic drug compounds. Hence, these cumbersome processes present challenges for conventional preparation methods across multiple manufacturing scales.

Despite several methods available for producing liposomes at laboratory scale, there are only a few methods that are used for commercial manufacture that can deliver liposomes with the required critical quality attributes. Of all the methods previously described, ethanol injection followed by extrusion is the most commonly used method of manufacture of large-scale parenteral liposomes. The reason is the reproducibility of liposome particle size and polydispersity index compared to other small-scale manufacturing techniques and the preference of using ethanol over chloroform.

Compared to conventional bulk methods, continuous-flow microfluidics offers several potential benefits over more conventional large-scale and batch processes to produce nanoparticles. Specifically, the ability to conduct reactions at scales comparable to the diffusion length with precise control of flow geometry and reaction conditions make it possible to avoid significant deviations in microenvironment and residence times, which can occur in larger systems (US 8,563,325 B1). Hence, continuous-flow microfluidics has emerged as a promising technology to alleviate some of the shortcoming of conventional bulk-scale liposome production methods for scalable liposome production with tunable control over the physical properties of the final product, particularly in terms of liposome size, size distribution, and lamellarity, allowing on-demand vesicle synthesis using a continuous-flow approach.

Continuous-flow focusing methods broadly can be categorized by device geometry, e.g., planar microfluidic, coaxial micro-/milli-fluidic focusing, and modified ethanol injection methods (US 6,843,942 B2) and their underlying physico-chemical mixing conditions, e.g., laminar or turbulent flow.

Whereas batch liposome preparation involves turbulent flows in a sequence of large fixed-volume reactors, microfluidic hydrodynamic flow-focusing (MHF) takes advantage of controllable laminar flow streams and short diffusion length scales in a small-volume process enabling continuous-flow synthesis of small uniform liposomes without the need for post-processing steps to decrease the size or polydispersity of the resulting liposome sample. In MHF-directed liposome production, a center fluid stream of solvated lipids in alcohol is sheathed between two oblique aqueous buffer streams focusing the center lipid stream into a narrow sheet with width defined by the ratio of flow rates between the aqueous and organic lipid streams enabling controlled diffusive mixing over length scales on the order of several hundred nanometers. The hydrodynamic focusing and controlled diffusion of solvent from the narrow lipid stream results in a sharp lipid solubility gradient that promotes rapid lipid self-assembly of unilamellar lipid vesicles of remarkably consistent size and low polydispersity that may be adjusted by changing the ratio of aqueous volumetric flow rates to alcohol volumetric flow rates and thereby the mixing conditions (US 9,198,645 B2).

Despite the progress in liposome size control with continuous-flow production methods, MHF methods have been constrained by the relatively low throughput and thereby prevented its applicability in bulk production (e.g., such as for large scale in-vivo studies and preclinical trials where larger volumes and higher concentrations are required). With the focus on increasing liposome throughput or production rate, modified microfluidic methods were explored, such as multiple MHF elements operating in parallel and vertical flow focusing (VFF). With respect to MHF operating in parallel it is desirable to minimize the degree of parallelization since slight variations in channel geometry and flow resistance across the flow focusing elements can degrade sample homogeneity. Furthermore, large numbers of parallel microfluidic elements can have a negative impact on the overall reliability of the continuous-flow system. In VFF devices, the lipid stream is focused by sheath flows above and below the lipid channel, rather than on the sides as in conventional MHF designs. The use of stacked channels oriented vertically within the microfluidic chip allows for the fabrication of channels with much higher aspect ratios, and a commensurate increase in maximum lipid flow rate. With respect to VFF the fabrication of high resolution and large aspect ratio features in VFF devices remains challenging due to the need for precise alignment between multiple flow paths within the focusing zone, since small misalignments can result in significant variations in the focusing geometry and resulting liposome populations. Furthermore, because VFF chip fabrication requires the bonding of five separate polymer layers to form the desired channel structures, maximum flow rates within the devices are dictated by the bond strength rather than laminar flow limits, thereby reducing maximum liposome production rates within the system.

For the purpose of particle generation, any simple 2-dimensional 2-or 3-stream laminar mixing is less than ideal for i) the planar (reacting) interface between the streams contacts the channel wall (i.e., floor and ceiling), tending to result in surface crystal nucleation that can lead to a loss of particle uniformity or even channel clogging, and ii) the parabolic velocity profile typical of pressure driven flow in a channel means that particles nucleating at different positions along the planar mixing interface will experience different velocities, residence times, and growth histories, tending to broaden the size distribution of resulting nanoparticles. Most mixing schemes suffer the wall nucleation problem, limiting their usefulness for continuous-flow nanoparticle production.

In order to overcome wall interactions coaxial-flow focusing techniques were developed, i.e., multi-barrel capillary-flow focusing (US 2013/0168885 A1) and coaxial turbulent jet mixing (US 10,632,072 B2), (Lim, et al.. Ultra-High Throughput Synthesis of Nanoparticles with Homogeneous Size Distribution Using a Coaxial Turbulent Jet Mixer. ACS Nano, 6056-6065 (2014)) where the lipid containing alcohol solution is passed through the core region and the aqueous solution is passed through the annular region thereby hydrodynamically focusing the core stream. While multi-barrel capillary-flow focusing technique can operate at higher flow rates than planar MHF devices due to the larger device dimensions it comes at the cost of liposome concentration; the very high volumetric flow rate ratios (i.e., the ratio of the aqueous flow rate to the solvent flow rate) required for the aqueous sheath flow to focus the central lipid-containing solvent stream greatly reduce liposome concentration and hence does not address lipid nanoparticle throughput per se. Thus, sufficient throughput and nanoparticle concentration have not been achieved utilizing traditional microfluidic techniques.

### Summary of the invention

Thus, the object of the present invention is to propose a novel device and a novel method for the formation of organic nanoparticles, with which the above-described drawbacks of the known devices and methods are completely overcome or at least greatly diminished.

The objectives of the present invention are achieved by a device for the formation of organic nanoparticles, comprising a cylindrical liquid feed line and a first annular liquid feed line, wherein the cylindrical feed line and the first annular feed line are coaxially arranged and the first annular feed line extends around the cylindrical feed line, the device comprising a first inlet for the introduction of a first liquid into the cylindrical feed line and a second inlet for the introduction of a second liquid into the first annular feed line, wherein the device comprises a second annular feed line and a third inlet for the introduction of a third liquid into the second annular feedline, wherein the second annular feed line is coaxially arranged with the cylindrical feed line and the first annular feed line, and wherein the second annular feed line extends around and beyond the cylindrical feed line and the first annular feed line.

Such a device allows for hydrodynamic focusing of a stream of an organic liquid comprising amphiphilic molecules sandwiched between two adjacent streams of aqueous solutions. For particle generation, the annular (reacting) interface between the aqueous streams does not contact tube walls within the vesicle formation zone, eliminating crystal nucleation that can lead to a loss of particle uniformity or even channel clogging. This coaxial device significantly improves the usefulness for continuous-flow nanoparticle production. Furthermore, the concentric arrangement means that particles nucleating along the annular mixing interface will experience a constant circumferential and near constant radial velocity (due to the narrow focusing stream width of the organic stream), residence time, and growth history, tending to narrow the size distribution of resulting nanoparticles. Furthermore, this arrangement allows for the encapsulation of a single or a mixture of active pharmaceutical ingredients (API), or a single or a mixture of APIs at two different concentrations, or two different single or mixtures of APIs dissolved in the aqueous streams, and a single or mixture of API co-dissolved in the lipid-containing organic stream. Thus, as lipids self-assemble into liposomes API dissolved in the aqueous streams is encapsulated in the aqueous interior of the forming liposomes, and API co-dissolved in the organic stream is sequestered into the lipid bilayer space.

In a first preferred embodiment of the present invention, the cylindrical feed line ends in a first liquid outlet and the first annular feed line ends in a second liquid outlet, and wherein the first liquid outlet and the second liquid outlet are positioned in a common plane.

This arrangement is favourable as it ensures that hydrodynamic focusing is initiated simultaneously at both interfaces between the organic stream and adjacent aqueous streams.

In another preferred embodiment of the present invention, the device comprises a third annular feed line and a fourth inlet for the introduction of a fourth liquid into the third annular feed line, wherein the third annular feed line is coaxially arranged with the cylindrical feed line, the first annular feed and the second annular feed line, wherein the third annular feed line is positioned between the first annular feed line and the second annular feed line, and wherein the second annular feed line extends beyond the third annular feed line.

This arrangement of three annular feed lines and one cylindrical feed line allows for splitting the aqueous stream into a central aqueous stream not containing API and an adjacent annular aqueous stream containing API, in which the first annular aqueous stream containing API is in the immediate vicinity of the mixing interface where encapsulation of API into lipid-vesicles occurs during lipid self-assembly. Thereby increasing the encapsulation efficiency by reducing the amount of non-encapsulated API. API is contained in the aqueous streams and/or the lipid containing organic stream. Furthermore, this arrangement allows for the encapsulation of a single or a mixture of APIs, or a single or a mixture of APIs at two different concentrations on both sides of the aqueous-organic mixing interface into the aqueous interior of the liposomes, and the sequestering of API dissolved in the organic stream into the lipid bilayer space during liposome formation.

In yet another preferred embodiment of the present invention, the device comprises a fourth annular feed line and a fifth inlet for the introduction of a fifth liquid into the fourth annular feed line, wherein the fourth annular feed line is coaxially arranged with the cylindrical feed line, the first annular feed line, the second annular feed line and the third annular feed line, wherein the fourth annular feed line is positioned between the second annular feed line and the third annular feed line, and wherein the second annular feed line extends beyond the fourth annular feed line.

This arrangement of four annular feed lines and one cylindrical feed line allows for one or two coaxial feeds of organic streams sheathed by adjacent aqueous streams containing a single or a mixture of API dissolved in both aqueous streams. Furthermore, it allows for different mixtures of API in the adjacent aqueous streams thereby producing combinations of liposomes encapsulating a first API or mixture of APIs and a second API or mixture of API into the aqueous interior of the produced liposomes and/or encapsulating a single or a mixture of APIs co-dissolved in the lipid-containing organic stream into the lipid-bilayer space of the produced liposomes. The feature of injecting two organic streams simultaneously allows for a further increase of liposome concentration and reduces the waste of non-encapsulated API compared to a single annular injection of organic liquid. Furthermore, in the advent when the reduction of API is paramount, a single organic stream is favoured in the third annular feed line and API-containing aqueous streams are sheathed to the immediate vicinity of the mixing interface through the first annular feed line and fourth annular feed line by adjacent aqueous streams without API through second annular feed line and the cylindrical feed line. Again, a single API or a mixture of APIs can be distributed between the first and fourth annular aqueous feed lines producing liposomes with varying content of encapsulated API species. Furthermore, depending on the chosen hydrodynamic focusing conditions for each organic stream (in the case of two organic streams) nanoparticle, especially liposomes, populations with similar or differing size and polydispersity can be achieved simultaneously in a continuous-flow mode.

In a further preferred embodiment of the present invention, the device comprises a cylindrical focusing element positioned inside the cylindrical feed line and coaxially arranged with the cylindrical feed line, wherein the focusing element extends beyond the cylindrical feed line.

This arrangement with an extended central focusing element results in comparable hydrodynamic focusing of the organic stream at a lower volumetric flow rate ratio of aqueous to organic liquid and hence increases the produced liposome concentration. Furthermore, the improved symmetry of the hydrodynamic focusing of the organic stream by the two adjacent aqueous streams improves, i.e. reduces, the polydispersity of the produced liposome population.

In another preferred embodiment of the present invention, the cylindrical feed line, the first annular feed line, the second annular feed line, the third annular feed line and the fourth annular feed line are formed by coaxially arranged conduits. This allows for a particularly simple manufacturing of this device. Furthermore, it is advantageous to provide for conduits that can be attached to one another by means of an attachment means, such as a snap couplings or threads. This allows for dissembling the device for cleaning or for the replacement of one or more conduits, for instance to adapt the diameter of the conduits to the desired nanoparticles size intended.

In yet another preferred embodiment of the present invention, the conduits are dispensing needles. Medical grade dispensing or hypodermic needles can be obtained from multiple manufacturers and hence allows the manufacturing of the device with standardized fluid connectors and the ability to sterilize the device. The wide availability of dispensing needles allows for economic and quick bench-top development obviating the need for special purpose clean room equipment and facilities. Furthermore, the wide range of available dispensing needle geometries allows for a multitude of combinations to tailor the coaxial device to one's specific needs.

In a further preferred embodiment of the present invention, the conduits comprise attaching means, for instance threads or snap couplings, for the attachment to one another. This allows for a quick assembly and disassembly of tubes and sterilization of all or individual parts of the coaxial mixing device. Furthermore, it provides a means for quick exchange of parts.

The objectives of the present invention are also achieved by a method for the formation of organic nanoparticles, comprising the steps of:
- providing a first liquid to a cylindrical feed line at a first fluid flow velocity,
- providing a second liquid to a first annular feed line at a second fluid flow velocity,
- providing a third liquid to a second annular feed line at a third fluid flow velocity, wherein one of the first liquid, second liquid or third liquid is an organic solution comprising amphiphilic molecules, wherein the other two liquids are a first aqueous solution and a second aqueous solution, and wherein the fluid flow velocity of the organic liquid comprising the amphiphilic molecules is lower than the fluid flow velocities of the first aqueous solution and the second aqueous solution,
- dispersing the amphiphilic molecules with the two adjacent aqueous solutions to the organic solution; and
- forming organic nanoparticles,
wherein the cylindrical feed line, the first annular feed line and the second annular feed line are coaxially arranged.

When the two liquid phases come into contact, the organic liquid rapidly diffuses into the aqueous phase and vice versa. The fluid flow velocities of the organic stream and aqueous streams can be adjusted to control the degree of hydrodynamic focusing and ultimately the liposome size. The concentric arrangement means that particle nucleating along the annular mixing interface will experience a constant circumferential and near constant radial velocity (due to the narrow focusing stream width of the organic stream), residence time, and growth history, tending to narrow the size distribution of resulting nanoparticles. Furthermore, this method allows for the encapsulation of a single API or mixture of APIs, or a single API or a mixture of APIs at two different concentrations, or two different APIs or mixtures of APIs dissolved in the aqueous stream into the aqueous interior of the liposome. Furthermore, it allows for sequestering a single API or mixture of APIs co-dissolved in the lipid-containing organic stream into the lipid-bilayer of the liposome. Furthermore, this method allows for continuous-flow nanoparticle formation foregoing the need for time-consuming size-altering post-processing steps, i.e. sonication or homogenization.

In a first preferred embodiment of the second aspect of the present invention, the method comprises the further step of providing a third aqueous solution to a third annular feed line at a fourth fluid flow velocity, wherein the third annular feed line is coaxially arranged with the cylindrical feed line, the first annular feed line and the second annular feed line, wherein the fourth aqueous fluid flow velocity is higher than the fluid flow velocity of the organic liquid comprising the amphiphilic molecules.

This allows for splitting the aqueous stream into a central aqueous stream and an adjacent annular aqueous stream containing a single API or a mixture of APIs, in which the first annular aqueous stream containing API is in the immediate vicinity of the mixing interface where encapsulation of API into the aqueous interior of the lipid-vesicle occurs during lipid self-assembly. Thereby increasing the encapsulation efficiency by reducing the amount of non-encapsulated API. Furthermore, this allows for the encapsulation of a single API or a mixture of APIs, or a single API or a mixture of APIs at two different concentrations, or two different APIs dissolved in the aqueous stream. The fluid flow velocities of the adjacent aqueous streams are generally higher than the fluid flow velocity of the organic streams. Furthermore, it allows for sequestering of a single API or a mixture of APIs co-dissolved in the lipid-containing organic stream into the lipid-bilayer of the liposome.

In another preferred embodiment of the second aspect of the present invention, the method comprises the further step of providing a second organic solution to a third annular feed line at a fifth fluid flow velocity, wherein the fourth annular feed line is coaxially arranged with the cylindrical feed line, the first annular feed line, the second annular feed line, and the third annular feed line, wherein the fifth fluid flow velocity is lower than the fluid flow velocities of the aqueous solutions.

With this embodiment two lipid-containing organic streams are sheathed between two adjacent aqueous streams, thereby facilitating the hydrodynamic focusing of two organic streams by three aqueous streams. This configuration increases the concentration and throughput of produced liposomes. The three aqueous streams can contain a single API or a mixture of APIs at a single concentration or at different concentrations, thereby providing a means to encapsulate various combinations of API into the interior of the formed liposomes. A single API or a mixture of APIs at a single concentration or at different concentrations can be co-dissolved into the lipid-containing organic streams and subsequently sequestered into the bilayer of the formed liposomes.

In a further preferred embodiment of the second aspect of the present invention, the method comprises the further step of providing a fourth aqueous solution to a fourth annular feed line at a sixth fluid flow velocity, wherein the fourth annular feed line is coaxially arranged with the cylindrical feed line, the first annular feed, the second annular feed line and the third annular feed line. In order to obtain hydrodynamical focusing of the organic solutions, the sixth fluid flow velocity shall be, in case of providing a fourth aqueous solution, larger than the fluid flow velocity of the organic liquids comprising amphiphilic molecules.

A higher fluid flow velocity of the sheathing aqueous streams with respect to the sheathed organic stream is advantageous and provides the means for hydrodynamic focusing and thereby rapid interdiffusion of the organic and aqueous streams to a critical condition where lipids are no longer soluble and thus self-assemble into liposomes. The method of using one organic stream and sheathing it with four aqueous streams, (that is two aqueous streams on each side of the organic stream) is advantageous when it is paramount to increase the encapsulation efficiency, i.e. the ratio of encapsulated API to non-encapsulated API. Since liposome formation and encapsulation of API occurs at the immediate organic-aqueous interface only the two aqueous streams immediately next to the organic stream need to contain a single API or a mixture of APIs at a single concentration or at different concentrations while the outer aqueous streams do not need to contain API. A single API or a mixture of APIs at a single concentration can be co-dissolved into the lipid-containing organic stream and subsequently sequestered into the bilayer of the formed liposomes

In yet another preferred embodiment of the second aspect of the present invention, the first aqueous solution, the second aqueous solution, the third aqueous solution, the fourth aqueous solution, the first organic solution, and/or the second organic solution comprise a reagent, wherein the reagent, i.e. API, can be a drug, a fluorescent molecule, amino acids, a protein, peptide, polymer, DNA, mRNA, and/or RNA.

Solubilizing a single API or a mixture of APIs at a single concentration or at different concentrations into the aqueous solutions adjacent to organic streams allows for the encapsulation of the solubilized API or mixture of APIs into the aqueous interior of the formed liposomes. Co-solubilizing a single API or a mixture of APIs in a single concentration or at different concentrations in the lipid-containing organic streams allows for sequestering of the co-solubilized API or mixture of APIs into the lipid-bilayer space of the formed liposomes.

In a further preferred embodiment of the second aspect of the present invention, the first organic solution and/or the second organic solution comprise phospholipids molecules.

Lipid-forming materials solubilized in an organic solvent and subsequent mixing with an aqueous solution thereby reducing the lipid-containing solvent to a solvent concentration below that where lipids are no longer soluble will result in the formation of liposomes.

In another preferred embodiment of the second aspect of the present invention, the Reynolds number at the first fluid flow velocity, the second fluid flow velocity, the third fluid flow velocity, the fourth fluid flow velocity, the fifth fluid flow velocity and the sixth fluid flow velocity is smaller than 2600, preferably smaller than 1200, advantageously smaller than 500, especially smaller than 300. The lower the surface roughness of the utilized tubing the higher the Reynolds number at which laminar flow conditions prevail. Preferably, the average fluid flow velocity of the organic solvent phase is 1-1000 mm/s, preferably 1-800mm/s, advantageously 1-500 mm/s, especially 1-300 mm/s for the aqueous solution and the average total fluid flow velocity in the vesicle formation zone 2 is 1-300 mm/s.

The fluid flow velocity difference between the organic stream and the adjacent aqueous streams defines the degree of hydrodynamic focusing. Laminar flow conditions in the coaxial continuous-flow device ensure predictable and controllable liposome formation and encapsulation of API in the aqueous interior of the produced liposomes and/or in the lipid-bilayer space of the produced liposomes.

Advantageously the method of the present invention, it carried out by means of a device according to the present invention.

### Brief description of the drawings

Figure 1 is a longitudinal cross-section of a device for the formation of organic nanoparticles according to a first preferred embodiment;
Figure 2 is a detailed view of section B of the vesicle formation zone of the device of Figure 1;
Figure 3 is a lateral cross-section along direction A-A of the device of Figure 1;
Figure 4a is a first perspective view of the results of a simulation of the hydrodynamic focusing of an annular organic flow stream with an adjacent concentric and central aqueous flow stream with the device of Figure 1;
Figure 4b is a second two-dimensional view of the results of a simulation of the hydrodynamic focusing of an annular organic flow stream with an adjacent concentric and central aqueous flow stream with the device of Figure 1;
Figure 5 is a longitudinal cross-section of a device for the formation of organic nanoparticles according to a second preferred embodiment;
Figure 6 is a detailed view of section B of the vesicle formation zone of the device of Figure 5;
Figure 7 is a lateral cross-section along direction A-A of the device of Figure 5;
Figure 8a shows a perspective view of the results of a simulation of the hydrodynamic focusing of an annular organic flow stream with one central and two adjacent concentric aqueous streams with the device of Figure 5;
Figure 8b shows a two-dimensional view of the results of a simulation of the hydrodynamic focusing of an annular organic flow stream with one central and two adjacent concentric aqueous streams with the device of Figure 5;
Figure 9 is a longitudinal cross-section of a device for the formation of organic nanoparticles according to a third preferred embodiment;
Figure 10 is a detailed view of section B of the vesicle formation zone of the device of Figure 9;
Figure 11 is a lateral cross-section along direction A-A of the device of Figure 9;
Figure 12a shows a perspective view of the results of a simulation of the hydrodynamic focusing of an annular organic flow stream with two adjacent concentric aqueous flow streams with the device of Figure 9;
Figure 12b shows a two-dimensional view of the results of a simulation of the hydrodynamic focusing of an annular organic flow stream with two adjacent concentric aqueous flow streams with the device of Figure 9;
Figure 13 is a longitudinal cross-section of a device for the formation of organic nanoparticles according to a fourth preferred embodiment;
Figure 14 is a detailed view of section B of the vesicle formation zone of the device of Figure 13;
Figure 15 is a lateral cross-section along direction A-A of the device of Figure 13;
Figure 16a shows a s perspective view of the results of a simulation of the hydrodynamic focusing of two annular organic streams alternating with three concentric aqueous streams with the device of Figure 13; and
Figure 16b shows a two-dimensional view of the results of a simulation of the hydrodynamic focusing of two annular organic stream alternating with three concentric aqueous flow streams with the device of Figure 13.

### Detailed description of preferred embodiments

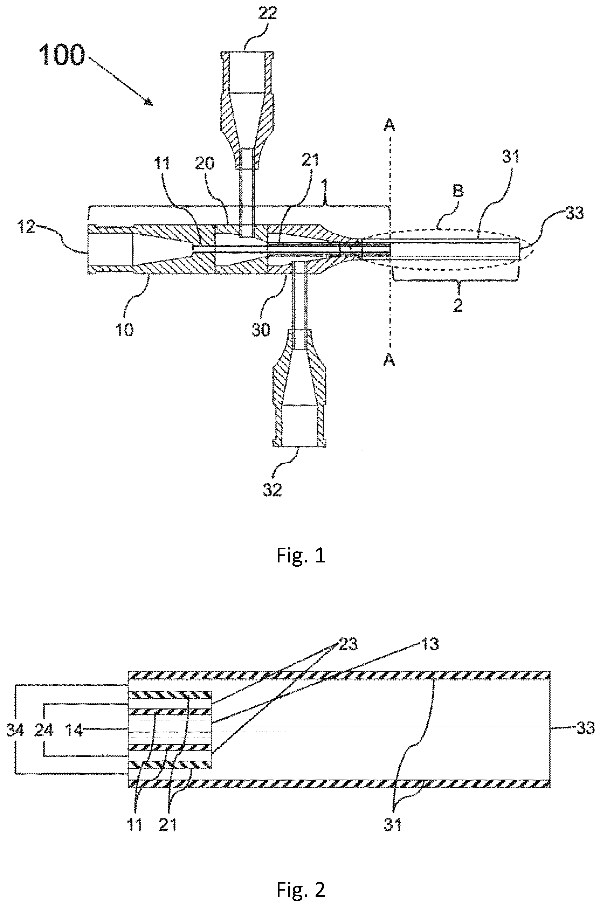

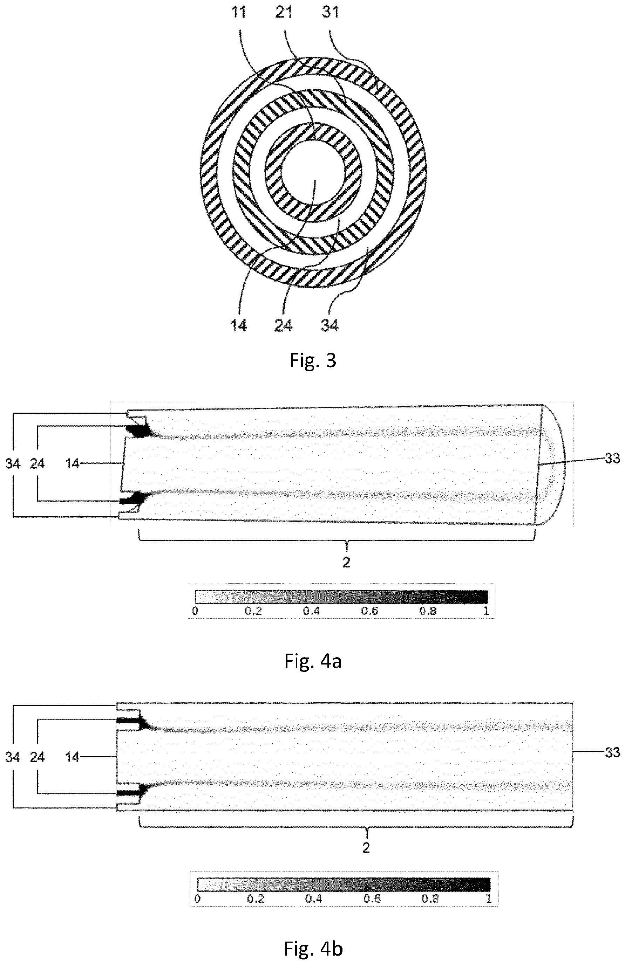

Figure 1 to 3 illustrate a device 100 for the formation of organic nanoparticles according to a first embodiment of the present invention. Figure 2 shows a detailed view of region (B) of device 100. Figure 3 shows a cross-sectional view along the direction A-A of continuous-flow device 100. The fluid introduction zone 1 comprises three coaxially arranged cylindrical conduits, 11, 21, 31 supported by their corresponding attaching means 10, 20, 30. The conduits 11, 21, 31 can be for instance stainless steel dispense needles, that are secured, i.e. threaded or snaped, into each other in a sequential manner resulting in a coaxial arrangement of the conduits 11, 21, and 31. Increasing in diameter from the innermost conduit 11 to the outermost conduit 31. The inner diameters of the conduits may vary from commercially available gauges 3 to 34, i.e. inner diameters ranging from approximately 0.05 mm (gauge 34) to 6 mm (gauge 3), and the wall thickness may vary among commercially available thicknesses from 0.025 mm to 0.5 mm.

As can be seen in Figure 3, the conduits 11, 21 and 31 form three feed lines, a cylindrical feed line 14, a first annular feed line 24 and a second annular feed line 34, wherein first annular feed line 24 is formed by the space between first conduit 11 and second conduit 21 and second annular feed line 34 by the space between the second conduit 21 and third conduit 31. A plurality of liquid inlets 12, 22, 32 serve as access points for the liquid to be inserted into the feed lines 14, 24, 34, in particular for introduction of organic and aqueous liquids. In the case where device 100 is made from commercially available dispensing needles, first dispensing needle has advantageously an inlet 12 coaxially arranged with the conduits 11, 21, 31 while the second and third dispensing needles are advantageously modified to provide fluid inlets 22 and 32 on the side of the device.

The first and second conduits 11, 21, and thus the cylindrical feed line 14 and the first annual feed lines 24, have terminal liquid outlets 13, 23 that are in a common plane. The outermost third conduit 31 extends the first and second conduits 11 and 21 and forms the second annular feed line 34 that exhibits terminal end 33 that is downstream or at a greater distance from terminal ends 13 and 23 (see Figure 2). Thus the second annular feed line 34 extends beyond the cylindrical feed line 14 and the first annular feed line 24. The conduits 11, 21, 31 may be secured concentrically with three circumferentially distributed equidistant nodules to each adjacent conduit (not shown).

Vesicle formation zone 2 comprises a length of outer conduits 31 and extends from first and second outlets 13, 23 to third outlet 33. The inner coaxial cylindrical conduits 11 and 21 protrude a distance of up to 20 mm, or other selected distance advantageously in common plane into the third conduit 31. The protrusion of the inner coaxial conduits into the third conduit is a function of the Reynolds number (Re) and approximately can be estimated to (0.6 + 0.05 x Re) x Dₕ, where Dₕ is the hydrodynamic diameter. The onset of the transition from laminar flow to turbulent flow starts at a Re of about 2600 according to general knowledge. However, depending on the surface roughness of the utilized tubes the transition from laminar to turbulent flow may occur for Re's as low as 300 to 500. In the embodiment shown in Figure 1, device 100 is configured and disposed for feeding, for instance, an annular organic stream from inlet 22 into parallel flowing annular sheathing aqueous streams being fed into inlets 12 and 32.

Device 100 provides for the formation of vesicles with a controlled proportion of organic, for instance alcohol, streams containing lipid-forming material, flowing through inlet 22 with respect to a volume of aqueous, i.e. polar, streams flowing through inlet 12 and 32 in vesicle formation zone 2. Aqueous stream inlet 12 may be configured for a first fluid flow velocity, aqueous stream inlet 32 may be configured for a second fluid flow velocity, and organic stream inlet 22 may be configured for a third fluid flow velocity. The fluid flow velocities of the aqueous solution are advantageously greater than or equal to the fluid flow velocity of the organic solution. The device is configured for a controlled and substantially uniform dispersion of an organic material in the organic stream at a plane perpendicular to vesicle formation zone 2.

A method for the formation of vesicles comprises flowing a first aqueous stream into cylindrical feed line 14, via inlet 12 and a second aqueous stream into second annular feed line 34, via inlet 32. An organic stream containing organic nanoparticles precursors as for instance lipid molecules is then fed into first annular feed line 24, via inlet 22 and thereby coaxially sheathed and hydrodynamically focused between the two adjacent aqueous streams through outlet 23. A lipid-forming material dissolved in the water-miscible organic stream diffusively mixes with the two adjacent aqueous streams in vesicle formation zone 2 and lipid vesicles are expelled from outlet 33. For example, an organic stream containing one or more organic molecules, such as amphiphilic molecules, or phospholipids and their derivatives, can be fed into vesicle formation zone 2 through inlet 22. An aqueous stream is fed into vesicle formation zone 2 through inlets 12 and 32. The organic molecules disperse throughout the co-flowing streams after the common plane of outlets 13 and 23 as lipid «rafts» and subsequently self-assemble spontaneously into liposomes in vesicle formation zone 2. Upon dispersion in the aqueous solution, the phospholipids form closed vesicles or liposomes, which can be characterized by spherical lipid bilayers substantially encapsulating an interior aqueous core. The sheathing of the organic stream with the adjacent concentric aqueous streams creates physico-chemical conditions in the aggregate co-flowing streams that are substantially symmetric about the annulus of the co-flowing streams. As the mutually miscible aqueous and organic streams disperse or diffusively mix the concentration of the lipid-containing organic solvent reduces to a critical condition where amphiphilic molecules are no longer soluble and thus self-assemble into lipid vesicles or liposomes.

Figures 4a and 4b show the results of a simulation illustrating the mutual dispersion of miscible fluids in vesicle formation zone 2 of the coaxial flow device for the formation of organic nanoparticles, in particular lipid vesicles, according to the embodiment of Figures 1 to 3. First annular feed line 24 is coaxially feeding a lipid-containing organic solution between two adjacent aqueous streams flowing from coaxial second annular feed line 34 and central cylindrical feed line 14. The stream of organic liquid is hydrodynamically focused between two sheathing aqueous streams into vesicle formation zone 2 and results in a device configured for controlled and substantially uniform dispersion of an organic material in the co-flowing streams, at a plane perpendicular to vesicle formation zone 2.

Figures 4a and 4b show in detail a numerical model of two aqueous, i.e. polar, liquid streams flowing through second annular feed line 32 and central cylindrical feed line 14 and an organic liquid stream flowing through first annular feed line 24, mixing, diffusing, or dispersing, just post second liquid outlet 23. In this gray scale model, it is shown that alcohol, represented by the dark annular longitudinal portion extending from first annular feed line 24, is hydrodynamically focused into a thin sheath and distributed along a channel diameter of outer sheath. The aqueous flow is represented by the white outer and central flow portion adjacent to the dark gray organic solvent stream. As can be seen with the controlled reduction of alcohol in the sandwiched annular organic stream, a controlled dispersion of alcohol is exhibited with the co-flowing streams. Figures 4a and 4b show that the outer flowing aqueous stream and the centrally flowing aqueous stream sheathes and hydrodynamically focuses the annular organic/alcohol stream. In this respect, a mutual diffusion of miscible fluids, in an alcohol stream and an aqueous stream, enables the assembly of vesicles at the solvent interface due to a change in amphiphile solubility. The lookup tables in Figure 4a and 4b represents the ethanol mole fraction.

The numerical model was generated with a finite-element numerical modeling software package that uses a laminar flow condition limit of the Navier-Stokes equations coupled with a convection-diffusion equation. An organic/aqueous mixture may have a viscosity that is greater than either liquid in pure form, therefore a plot of viscosity as a function of alcohol concentration in water may have a roughly parabolic shape with a maxima at ∼60% ethanol in water. Hence, the diffusion coefficients may have a minima at the same concentration conditions. Any heats of mixing were neglected as they may be negligible.

When the organic and aqueous liquid phases come into contact, the organic phase and aqueous phase may rapidly diffuse into one another. The fluid flow velocities of the organic may be adjusted to 1-150 mm/s and aqueous streams may be adjusted to 1-800 mm/s to control the degree of hydrodynamic focusing and ultimately the liposome size. The lipids self-assemble where the concentration of the organic phase containing the lipid or lipid-forming materials and the aqueous composition is at a critical condition where lipids are no longer soluble and thus self-assemble into liposomes.

One can control the physical characteristics of the resultant liposome preparation by altering the ratio of the fluid flow velocities of the aqueous liquid compared to the organic lipid-containing liquid sandwiched between the aqueous containing sheathing feed lines. This results in a decrease or increase in both the mean size and polydispersity of liposome diameter. For example, increasing the fluid flow velocity ratio (i.e., aqueous fluid flow velocity to organic fluid flow velocity) and thereby increasing the degree of hydrodynamic focusing, which in turn decreases the stream width of the lipid-containing organic stream, decrease both polydispersity and size of the liposomes formed.

A useful characteristic of liposomes is their ability to encapsulate ionic molecules from a surrounding polar/aqueous medium and sequester nonpolar molecules into the lipid-bilayer of the lipid vesicles or liposomes from an organic medium. Thus, the present disclosure includes embodiments wherein a reagent is co-dissolved in the organic liquid containing a composition of lipids or lipid-forming materials and/or in the aqueous composition and at least a portion of the reagents encapsulated (or sequestered into the envelop of the organic nanoparticles) in the liposomes. Examples of reagents which may be encapsulated in liposomes as part of the above-described methods include small molecules (for example, drugs, fluorescent molecules, amino acids) and large molecules (for example, proteins, peptides, polymers, DNA, mRNA, and RNA).

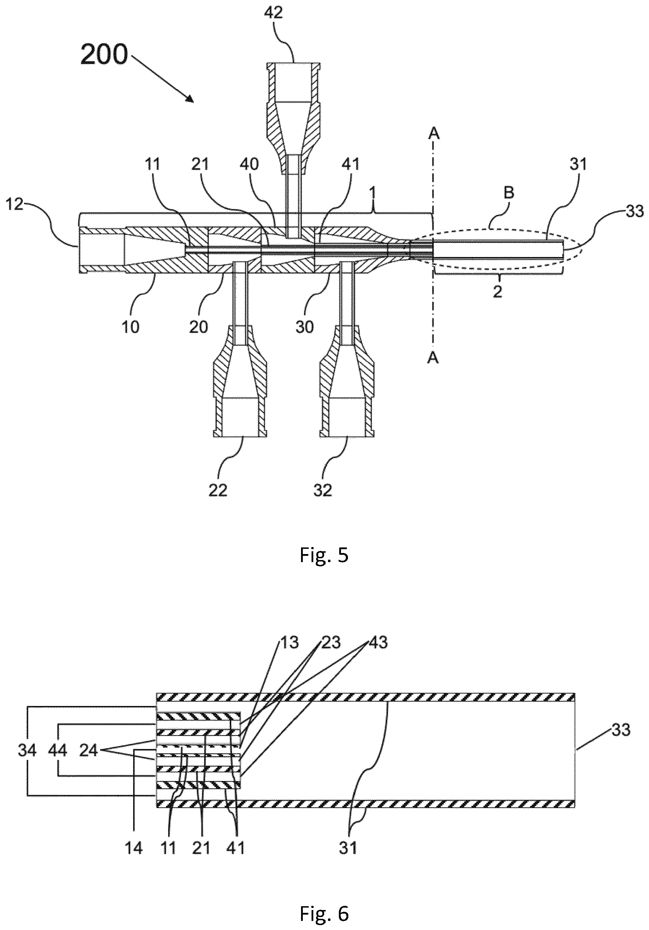

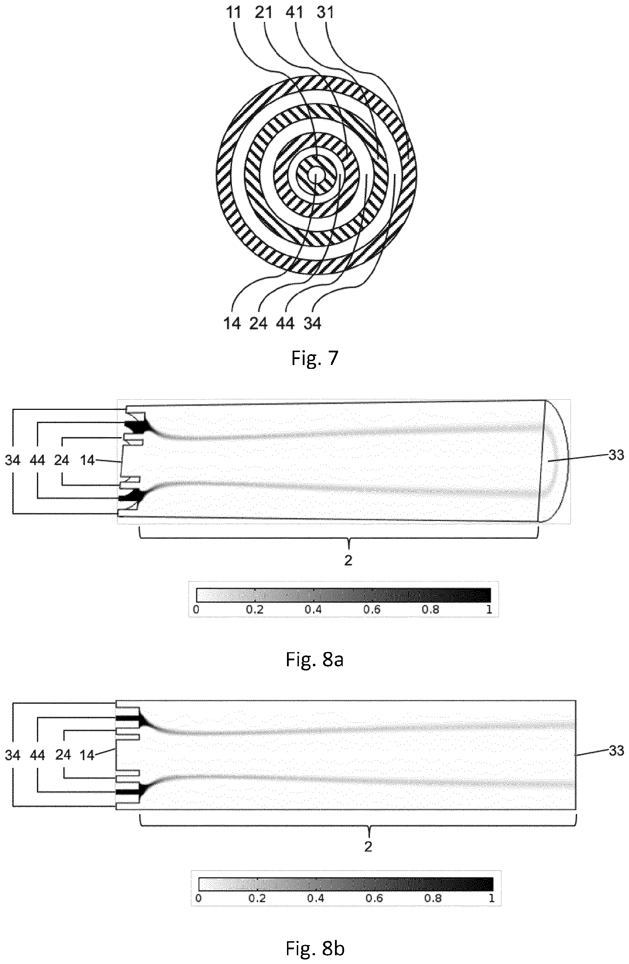

Figures 5 to 7 present a device 200 for the formation of organic nanoparticles according to a second embodiment of the present invention. Figure 6 shows a detailed view of region (B) of device 200. Figure 7 shows a cross-sectional view along the direction A-A of flow device 200. As device 100, device 200 has a fluid introduction zone 1 and a vesicle formation zone 2.

Device 200 is similar to device 100 but comprises a fourth cylindrical conduit 41 coaxially arranged with the first 11, second 21 and third conduit 31. As for device 100, device 200 can be assembled from multiple stainless steel dispense needles, that are secured, i.e., threaded or snaped, into each other in a sequential manner resulting in a coaxial arrangement of hypodermic tubes 11, 21, 31, and 41, respectively, increasing in diameter from the innermost conduit 11 to the outermost conduit 31. The inner diameters of the conduits may vary from commercially available gauges 3 to 34, i.e. inner diameters ranging from approximately 0.05 mm (gauge 34) to 6 mm (gauge 3), and the wall thickness may vary among commercially available thicknesses from 0.025 mm to 0.5 mm. As can been seen in Figure 7, the conduits 11, 21, 31 and 41 form four feed lines, a cylindrical feed line 14, a first annular feed line 24, a second annular feed line 34 and a third annular feed line 44, wherein the first annular feed line 24 is formed by first 11 and second 21 conduits, the second annular feed line 34 by the third conduit 31 and the fourth conduit 41 and the third annular feed 44 line by the second 21 and fourth conduit 41. A plurality of liquid feed inlets 12, 22, 32, 42 serve as access points for the organic and aqueous liquids for their introductions into the feed lines 14, 24, 34, 44. In case the device is assembled from dispensing needles, the first needle has advantageously an inlet 12 in line with the conduit 11, while the other dispensing needles are advantageously modified to provide with side access inlet 22, 32, and 42. The inner conduits 11, 21, 41 may each have terminal ends 13, 23, and 43 that protrude a distance of up to 20 mm, or other selected distance, advantageously in common plane. into the outermost conduit 31. The conduits may be secured concentrically with three circumferentially distributed equidistant nodules to each adjacent conduit.

In one embodiment of a method for the formation of organic nanoparticles, an organic stream may be introduced, for instance by means of a pump, into third annular feed line 44, and advantageously with a second, third, and fourth pump, an aqueous stream may be introduced into conduits of the cylindrical feed line 14, the first annular feed line 24, and the second annular feed line 34. Figures 8a and 8b show the results of simulations illustrating the mutual dispersion of miscible fluids in vesicle formation zone 2 of the coaxial flow device for the formation of organic nanoparticles, in particular lipid vesicles, according to this embodiment. The simulations of Figures 8a and 8b were performed with the same constraints as the simulation shown in Figures 4a and 4b.

In another embodiment, advantageously using a first pump and a second pump, an organic stream may be introduced into the cylindrical feed line 14 and the third annular feed line 44, and advantageously with a third and fourth pump, a first aqueous stream may be introduced into first annular feed line 24 and a second aqueous stream into second annular feed line 34.

With device 200 it is possible to create annularly symmetric physico-chemical conditions of fluids flowing into vesicle formation zone 2 and allows for substantially monodisperse preparations of lipid vesicles. At vesicle formation zone's 2 terminus, or outlet 33, a fluid containing vesicles may be collected and ready for use, further manipulation, or analysis.

Coaxial flow device 200 provides for the formation of vesicles with a controlled proportion of organic/alcohol streams containing lipid-forming material, flowing through conduits 11 and 41 with respect to a volume of polar/aqueous streams flowing through conduits 21 and 31 in vesicle formation zone 2. This configuration results in a higher concentration of vesicles, which may be desirable.

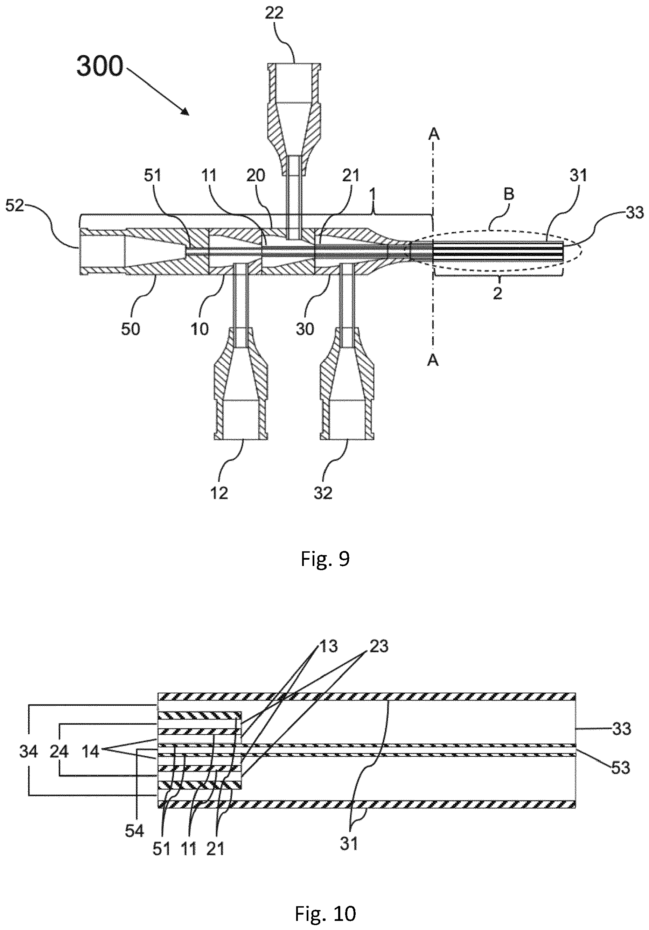

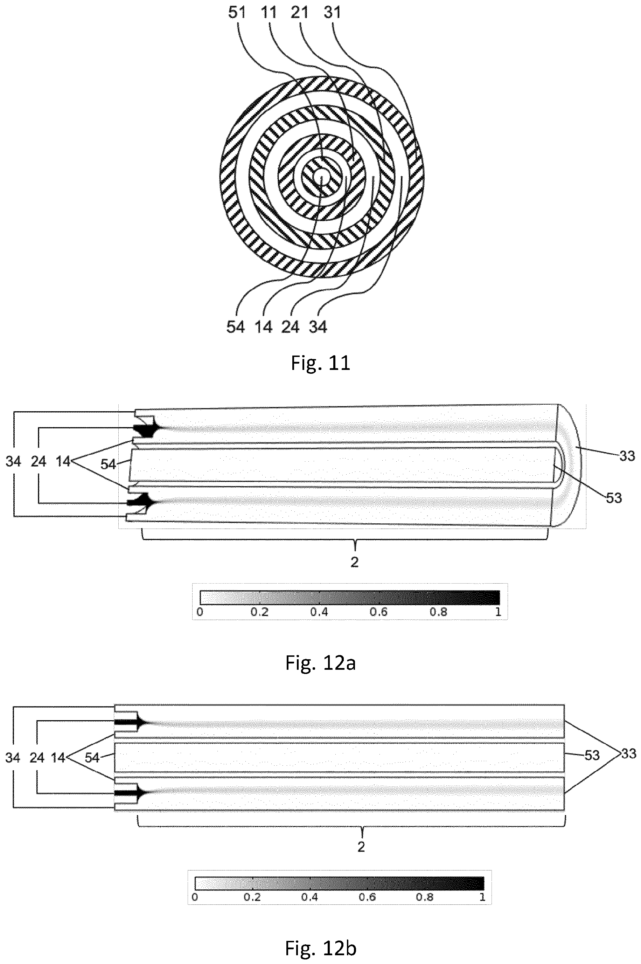

Figures 9 to 11 illustrate a device 300 for the formation of organic nanoparticles according to a third embodiment of the present invention. Figure 10 shows a detailed view of region (B) of flow device 300. Figure 11 shows a cross-sectional view of four coaxial conduits along direction A-A of flow device 300. Device 300 has a fluid introduction zone 1 and a vesicle formation zone 2.

Device 300 is similar to device 100, therefore only the differences between these two embodiments are discussed here. Device 300 comprises within the first conduit 11 a cylindrical and to the other conduits 21, 31 coaxially arranged focusing element 51. In the embodiment of Figure 9 to 11, the focusing element takes the form of a cylindrical conduit 51 similar to the first conduit 11 but with a smaller diameter. It is important to note, that the focusing element could also be a simple rod of a diameter smaller than the diameter of the first conduit. The presence of the focusing element enables to direct the stream of aqueous, i.e. polar, flow stream fed into feed line 14 in the direction of the organic flow fed into first annular feed line 24 and allows for a stronger hydrodynamic focusing of the organic flow at a lower aqueous volume. While in Figure 10 the outlet 53 of conduit 51 is positioned in the same plane at the liquid outlet 33, it could be provided at a different position inside or outside the third conduit 31, depending of the focusing effect sought. It is important to note that a focusing element 51, could be provided in every embodiment of a device or method for the formation of organic nanoparticles according to the present invention.

In one embodiment of the method of formation of organic nanoparticles according to present invention, an organic stream may be introduced into first annular feed line 24, and a first aqueous stream may be introduced into the cylindrical feed line 14 and a second aqueous stream into the second annular feed line 34. In another embodiment, an aqueous stream or a gaseous stream may be introduced into conduit 51. A gaseous stream may enhance the evaporation of residual organic solvent in the collected sample. In another embodiment conduit 51 may be plugged to prevent any flow of material into or out of outlet 53.

With device 300 it is possible to create annularly symmetric physico-chemical conditions of fluids flowing into vesicle formation zone 2 and allow for substantially monodisperse preparations of lipid vesicles. At vesicle formation zone's 2 terminus, or liquid outlet 33, a fluid containing vesicles may be collected and ready for use, further manipulation, or analysis. Similar to the device 100 and 200, the first 11, second 21 and third 31 conduits, as well as the focusing element 51, can, advantageously, take the form of dispensing needle that are attached together by means of their supporting means. As shown in Fig. 11, the different feed lines 14, 24, 34 are formed by the coaxially arranged conduits 11, 21, 31.

Figures 12a and 12b show the results of simulations illustrating the mutual dispersion of miscible fluids in vesicle formation zone 2 of the coaxial flow device for the formation of organic nanoparticles, in particular lipid vesicles, by means of device 300. As can be seen in these Figures, thanks to the presence of the hydrodynamical focusing element 51, the hydrodynamic focusing exhibits a stronger symmetry and comparable hydrodynamic focusing and hence dilution of the organic solvent can be accomplished at lower volumetric flow rate ratios (i.e. the volumetric flow rate of aqueous phase to organic phase). The simulations of Figures 12a and 12b were performed with the same constraints as the simulation shown in Figures 4a and 4b.

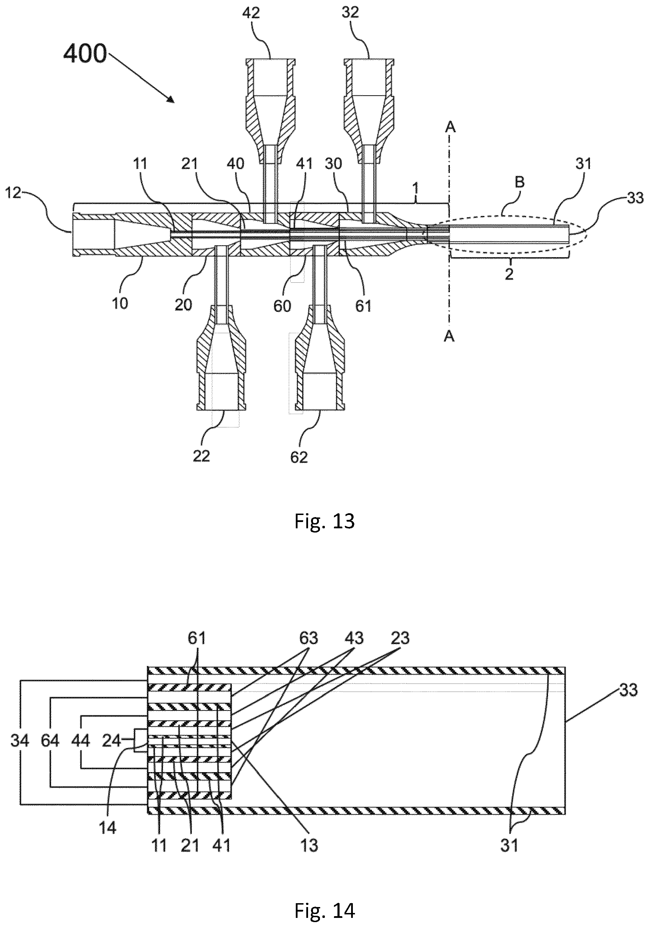

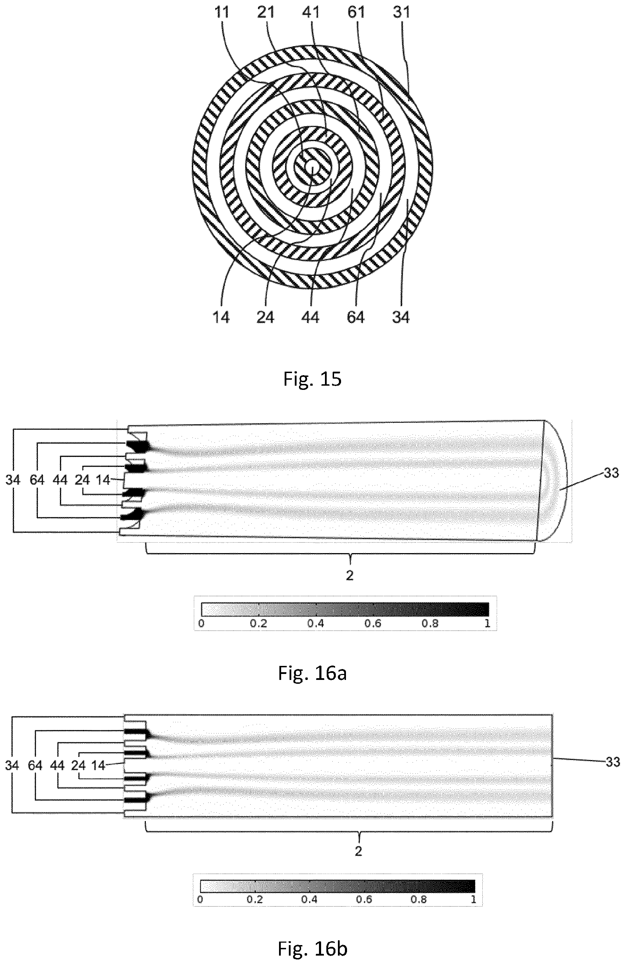

Figures 13 to 15 illustrate a device 400 for the formation of organic nanoparticles according to a fourth embodiment of the present invention. Figure 14 shows a detailed view of region (B) of flow device 400. Figure 15 shows a cross-sectional view of five coaxial conduits along direction A-A of flow device 400. Device 400 has a fluid introduction zone 1 and a vesicle formation zone 2.

Device 400 is similar to device 200 but comprises a fifth cylindrical conduit 61 coaxially arranged with the first 11, second 21, third 31, and fourth 41 conduits. As for device 200, device 400 can be assembled from multiple dispense needles, advantageously made out of stainless steel, that are secured, i.e., threaded or snaped, into each other in a sequential manner resulting in a coaxial arrangement of hypodermic tubes 11, 21, 31, 41, and 61, respectively. Increasing in diameter from the innermost conduit 11 to the outermost conduit 31. The inner diameters of the conduits may vary from commercially available gauges 3 to 34, i.e. inner diameters ranging from approximately 0.05 mm (gauge 34) to 6 mm (gauge 3), and the wall thickness may vary among commercially available thicknesses from 0.025 mm to 0.5 mm. As can been seen in Figure 13, the conduits 11, 21, 31, 41, and 61 form five feed lines, a cylindrical feed line 14, a first annular feed line 24, a second annular feed line 34, a third annular feed line 44, and a fourth annular feed line 64, wherein the first annular feed line 24 is formed by first 11 and second 21 conduits, the second annular feed line 34 by the third conduit 31 and the fifth conduit 61, the third annular feed line 44 by the second conduit 21 and fourth conduit 41, and the fourth annular feed line 64 by the fourth conduit 41 and fifth conduit 61. A plurality of liquid feed inlets, 12, 22, 32, 42, 62 serve as access points for the organic and aqueous liquids for their introductions into the feed lines, 14, 24, 34, 44, 64. In case the device is assembled from dispensing needles, the first needle has advantageously an inlet 12 in line with the conduit 11, while the other dispensing needles are advantageously modified to provide with side access inlet 22, 32, 42, and 62. The inner conduits 11, 21, 41, 61 may each have terminal ends 13, 23, 43, and 63 that protrude a few millimeters, or other selected distance, advantageously in common plane, into the outermost conduit 31. The conduits may be secured concentrically with three circumferentially distributed equidistant nodules to each adjacent conduit.

In one embodiment of a method for the formation of organic nanoparticles, an organic stream may be introduced, for instance by means of a pump, into fourth annular feed line 64, and advantageously with a second, third, fourth and fifth pump, aqueous streams may be introduced into conduits of the cylindrical feed line 14, the first annular feed line 24, the third annular feed line 44, and the second annular feed line 34. In another embodiment, advantageously using a first pump and a second pump, organic streams may be introduced into the first annular feed line 24 and the fourth annular feed line 64, and advantageously with a third, fourth and fifth pump, aqueous streams may be introduced into conduits of the cylindrical feed line 14, the third annular feed line 44, and the second annular feed line 34. In another embodiment, advantageously using a first pump, an organic stream may be introduced into the first annular feed line 24, and advantageously with a second, third, fourth and fifth pump, aqueous streams may be introduced into conduits of the cylindrical feed line 14, the third annular feed line 44, the fourth annular feed line 64, and the second annular feed line 34. In another embodiment, advantageously using a first pump and a second pump, organic streams may be introduced into conduits of the central feed line 14 and third annular feed line 44, and and advantageously with a third, fourth and fifth pump, aqueous streams may be introduced into conduits of first annular feed line 24, the fourth annular feed line 64, and the second annular feed line 34.

Figures 16a and 16b show the results of a representative simulation illustrating the mutual dispersion of miscible fluids in vesicle formation zone 2 of the coaxial flow device for the formation of organic nanoparticles, in particular lipid vesicles, according to one embodiment, where two organic streams are introduced. The simulations of Figures 16a and 16b were performed with the same constraints as the simulation shown in Figures 4a and 4b.

With device 400 it is possible to create annularly symmetric physico-chemical conditions of fluids flowing into vesicle formation zone 2 and thus substantially monodisperse preparations of lipid vesicles. At vesicle formation zone's 2 terminus, or outlet 33, a fluid containing lipid vesicles may be collected and ready for use, further manipulation, or analysis. Similarly to device 100, 200, and 300 the first conduit 11, second conduit 21, third conduit 31, fourth conduit 41, and fifth conduit 61, can, advantageously, take the form of dispensing needle that are attached together by means of their supporting means. As shown in Fig. 13, the various feed lines 14, 24, 34, 44, and 64 are formed by the coaxially arranged conduits 11, 21, 31, 41, 61.

Coaxial flow device 400 provides for the formation of vesicles with a controlled proportion of organic, for instance alcohol, streams containing lipid-forming material, flowing through conduits 41 with respect to a volume of aqueous. i.e. polar, streams flowing through conduits 11, 21, 61, and 31 in vesicle formation zone 2. This configuration results in a lower concentration of vesicles but optimized consumption of API, which may be desirable. Furthermore, coaxial flow device 400 provides for the formation of vesicles with a controlled proportion of organic, for instance alcohol, streams containing lipid-forming material, flowing through conduits 21 and 61 with respect to a volume of aqueous, i.e. polar, streams flowing through conduits 11, 41, and 31 in vesicle formation zone 2. This configuration results in a higher consumption of API but also a higher concentration of vesicles, which may be desirable. Furthermore, coaxial flow device 400 provides for the formation of vesicles with a controlled proportion of organic, for instance alcohol, streams containing lipid-forming material, flowing through conduits 41 and 11 with respect to a volume of aqueous, i.e. polar, streams flowing through conduits 21, 31, and 61 in vesicle formation zone 2. This configuration results in a lower consumption of API but also a higher concentration of vesicles, which may be desirable. Finally, coaxial flow device 400 provides for the formation of vesicles with a controlled proportion of organic, for instance alcohol, streams containing lipid-forming material, flowing through feed lines 24 with respect to a volume of aqueous, i.e. polar, streams flowing through feed lines 14, 44, 64, and 34 in vesicle formation zone 2. This configuration results in a lower consumption of API but also a lower concentration of vesicles, which may be desirable.

In all embodiments of the method for the formation of organic nanoparticles according to the present invention, the aqueous composition can for instance be an aqueous buffer solution, particularly a phosphate buffered saline solution, phosphate buffer, TRIS buffer, HEPES buffer, or another suitable and pharmaceutically acceptable buffer, optionally with customary additives, such as preservatives, fragrances, colorants and the like, that are preferably used as carrier medium of the polar phase, both for cosmetic and pharmaceutical preparations, in particular those for medical applications. In one embodiment, phosphate buffered saline (Sigma Aldrich) solution (10 mM, pH 7.4) can be used as the hydration buffer. All fluids (solvent and buffer) shall be passed through 0.22 µm syringe filters (Millipore Corp., New Bedford, MA) before being introduced to the multi-coaxial continuous-flow device.

The lipid or lipid-forming materials used in the conduits to make liposomes include all known materials for liposome formation. Examples of useful materials include combinations of phospholipid molecules and cholesterol. Particularly preferred are combinations of 1,2-Dimyristoyl-sn-glycero-3-phosphocholine, cholesterol, and dicetylphosphate. These materials can for instance be provided in a solvent that will dissolve the lipid or lipid-forming materials. The solvent can also be water-miscible in order to diffuse or disperse into the aqueous composition. Examples of useful solvents include alcohols, such as isopropanol, methanol or ethanol. The lipids or lipid-forming materials may be provided in the solvent at a concentration of approximately 5 mM to 50 mM. The aqueous composition may be an aqueous buffer solution, particularly a phosphate buffered saline solution, phosphate buffer, TRIS buffer or HEPES buffer. In one method embodiment, 1,2-Dimyristoyl-sn-glycero-3-phosphocholine, cholesterol (Avanti Polar Lipids, Alabaster, AL), and dicetylphosphate (Sigma Aldrich, St. Louis, MO) can be mixed in chloroform (Mallinckrodt Baker Inc., Phillipsburg, NJ) at a molar ratio of 5:4:1. Each lipid mixture can be prepared in a glass scintillation vial when purging with nitrogen (N₂), and then stored in a vacuum desiccator for at least 24 h for complete solvent removal. The desiccated lipid mixture may be rehydrated in anhydrous ethanol (Sigma Aldrich) for a total lipid concentration ranging from 5 mM to 20 mM. Phosphate buffered saline (Sigma Aldrich) solution (10 mM, pH 7.4) may be used as the hydration buffer. All fluids (solvent and buffer) shall be passed through 0.22 µm syringe filters (Millipore Corp., New Bedford, MA) before being introduced to the multi-coaxial continuous-flow device. The temperatures of the liquids are dependent on the utilized amphiphilic molecules, for instance phospholipid molecules. The temperature shall range from 20°C to 36°C.

When the liquid phases come into contact, the organic solvent phase and aqueous phase may rapidly diffuse into one another. The fluid flow velocities of the solvent and aqueous streams can be adjusted to control the degree of hydrodynamic focusing and ultimately the liposome size. The lipids self-assemble where the concentration of the solvent phase containing the lipid or lipid-forming materials and the aqueous composition is at a critical condition where lipids are no longer soluble and thus self-assemble into liposomes. One can control the liposome size by altering the ratio of the fluid flow velocities in the sheathing inlet conduits compared to the sandwiched lipid-containing inlet conduit. This results in a decrease or increase in both the mean and range (polydispersity) of liposome diameters. Thus, by tuning of the fluid flow velocities in the inner and outer parallel flow streams, the physical characteristics of the resultant liposome preparation can be changed or controlled.

In all embodiments, the conduits can advantageously be made out of stainless steel, titanium, or plastics. The inner diameters of the conduits may vary from commercially available gauges 3 to 34, i.e. inner diameters ranging from approximately 0.05 mm (gauge 34) to 6 mm (gauge 3), and the wall thickness may vary among commercially available thicknesses from 0.025 mm to 0.5 mm. The tube length of the outer conduits encompassing the formation zone 2 may vary from commercially available length from 20 mm to 300 mm.

The flow behavior of the mixed stream is preferably laminar. The liquids shall have a Reynolds number smaller than 2600, preferably smaller than 1200, advantageously smaller than 500, especially smaller than 300.

The liposome self-assembly method described herein can be used to provide liposomes for in vivo applications and for on-demand drug encapsulation and delivery and may be scaled up or down providing low throughput devices or larger production scale devices.

Finally, it should be pointed out that the foregoing has outlined one pertinent non-limiting embodiment. It will be clear to those skilled in the art that modifications to the disclosed non-limiting embodiment can be done without departing from the spirit and scope thereof. As such, the described non-limiting embodiments ought to be considered merely illustrative of some of the more prominent features and applications. Other beneficial results can be realized by applying the non-limiting embodiments in a different manner or modifying them in ways known to those familiar with the art.

## Claims

1. A device (100, 200, 300, 400) for the formation of organic nanoparticles, comprising a cylindrical liquid feed line (14) and a first annular liquid feed line (24), wherein the cylindrical feed line (14) and the first annular feed line (24) are coaxially arranged and the first annular feed line (24) extends around the cylindrical feed line (14), the device comprising a first inlet (12) for the introduction of a first liquid into the cylindrical feed line (14) and a second inlet (22) for the introduction of a second liquid into the first annular feed line (24),
**characterized in that**,
the device (100, 200, 300, 400) comprises a second annular feed line (34) and a third inlet (32) for the introduction of a third liquid into the second annular feedline (34), wherein the second annular feed line (34) is coaxially arranged with the cylindrical feed line (14) and the first annular feed line (24), and wherein the second annular feed line extends around and beyond the cylindrical feed line (14) and the first annular feed line (24).

2. Device according to claim 1, wherein the cylindrical feed line (14) ends in a first liquid outlet (13) and the first annular feed line ends in a second liquid outlet (23), and wherein the first liquid outlet (13) and the second liquid outlet (23) are positioned in a common plane (A-A).

3. Device according to any one of the claims 1 or 2, comprising a third annular feed line (44) and a fourth inlet (42) for the introduction of a fourth liquid into the third annular feed line (44), wherein the third annular feed line (44) is coaxially arranged with the cylindrical feed line (14), the first annular feed line (24) and the second annular feed line (34), wherein the third annular feed line is positioned between the first annular feed line (24) and the second annular feed line (34), and wherein the second annular feed line (34) extends beyond the third annular feed line (44).

4. Device according to any one of the preceding claims, comprising a fourth annular feed line (64) and a fifth inlet (62) for the introduction of a fifth liquid into the fourth annular feed line (64), wherein the fourth annular feed line (64) is coaxially arranged with the cylindrical feed line (14), the first annular feed (24), the second annular feed line (34) and the third annular feed line (44), wherein the fourth annular feed line (64) is positioned between the second annular feed line (34) and the third annular feed line (44), and wherein the second annular feed line (34) extends beyond the fourth annular feed line (64).

5. Device according to any of the preceding claims, comprising a cylindrical focusing element (51) positioned inside the cylindrical feed line (14) and coaxially arranged with the cylindrical feed line (14), wherein the focusing element extends beyond the cylindrical feed line (14).

6. Device according to any of the preceding claims, wherein the cylindrical feed line (14), the first annular feed line (24), the second annular feed line (34), the third annular feed line (44) and the fourth annular feed line (64) are formed by coaxially arranged conduits (11, 21, 31, 41, 61).

7. Device according to claim 6, wherein the conduits (11, 21, 31, 41, 61) are dispensing needles.

8. Device according to any of the claims 6 or 7, wherein the conduits comprise (11, 21, 31, 41, 61) attaching means (10, 20, 30, 40, 60), for instance threads or snap couplings, for the attachment to one another.

9. Method for the formation of organic nanoparticles, comprising the steps of:
- providing a first liquid to a cylindrical feed line (14) at a first fluid flow velocity,
- providing a second liquid to a first annular feed line (24) at a second fluid flow velocity,
- providing a third liquid to a second annular feed line (34) at a third fluid flow velocity, wherein one of the first liquid, second liquid or third liquid is an organic solution comprising amphiphilic molecules, wherein the other two liquids are a first aqueous solution and a second aqueous solution, and wherein the fluid flow velocity of the liquid comprising the amphiphilic molecules is lower than the fluid flow velocities of the first aqueous solution and the second aqueous solution,
- dispersing the amphiphilic molecules with the two adjacent aqueous solutions to the organic solution; and
- forming organic nanoparticles,
wherein the cylindrical feed line (14), the first annular feed line (24) and the second annular feed line (34) are coaxially arranged.

10. Method according to claim 9, comprising the further step of providing a third aqueous solution to a third annular feed line (44) at a fourth fluid flow velocity, wherein the third annular feed line (44) is coaxially arranged with the cylindrical feed line (14), the first annular feed line (24) and the second annular feed line (34), wherein the fourth fluid flow velocity is higher than the fluid flow velocity of the liquid comprising the amphiphilic molecules.

11. Method according to claim 9, comprising the further step of providing a second organic solution to a third annular feed line (44) at a fifth fluid flow velocity, wherein the third annular feed line (44) is coaxially arranged with the cylindrical feed line (14), the first annular feed line (24) and the second annular feed line (34), wherein the fifth fluid flow velocity is lower than the fluid flow velocity of the aqueous solution.

12. Method according to any one of the claims 9 to 11, comprising the further step of providing a fourth aqueous solution to a fourth annular feed line (44) at a sixth fluid flow velocity, wherein the fourth annular feed line (64) is coaxially arranged with the cylindrical feed line (14), the first annular feed (24), the second annular feed line (34) and the third annular feed line (44).

13. Method according to any one of the claims 9 to 12, wherein the first aqueous solution, the second aqueous solution, the third aqueous solution, the fourth aqueous solution, the first organic solution and/or the second organic solution comprise a reagent, wherein the reagent can be a drug, a fluorescent molecule, amino acids, a protein, peptide, polymer, DNA, mRNA, and/or RNA.

14. Method according to any one of the claims 9 to 13, wherein the first organic solution and/or the second organic solution comprise phospholipid molecules.

15. Method according to any one of the claims 9 to 14, wherein the Reynolds number of the first fluid flow velocity, the second fluid flow velocity, the third fluid flow velocity, the fourth fluid flow velocity, the fifth fluid flow velocity and the sixth fluid flow velocity is smaller than 2600, preferably smaller than 1200, advantageously smaller than 500, especially smaller than 300.
